(19) Europäisches Patentamt
European Patent Office
Office européen des brevets

(11) Publication number : 0 618 223 A2

(12) # EUROPEAN PATENT APPLICATION

(21) Application number : 94810137.3

(22) Date of filing : 03.03.94

(51) Int. Cl.[5] : C07K 5/02, C07K 5/06, A61K 37/02

(30) Priority : 08.03.93 GB 9304686
22.04.93 GB 9308328
16.03.93 GB 9305382
22.04.93 GB 9308329
16.03.93 GB 9305386
22.04.93 GB 9308330

(43) Date of publication of application : 05.10.94 Bulletin 94/40

(84) Designated Contracting States : AT BE CH DE DK ES FR GB GR IE IT LI LU NL PT SE

(71) Applicant : SANDOZ LTD.
Lichtstrasse 35
CH-4002 Basel (CH)
(84) AT BE CH DE DK ES FR GB GR IE IT LI LU NL PT SE

(71) Applicant : SANDOZ-PATENT-GMBH
Humboldtstrasse 3
D-79539 Lörrach (DE)
(84) DE

(71) Applicant : SANDOZ-ERFINDUNGEN
Verwaltungsgesellschaft m.b.H.
Brunner Strasse 59
A-1235 Wien (AT)
(84) AT

(72) Inventor : Heng, Richard
Landoltstrasse 63
CH-3006 Bern (CH)
Inventor : Leutwiler, Albert
Reiderberg 351
CH-3178 Bösingen (CH)
Inventor : Révész, Laszlo
ob dem Fichtenrain 7
CH-4106 Therwil (CH)
Inventor : Wüthrich, Hans-Jürg
Gurtenstrasse 5
CH-3122 Kehrsatz (CH)

(54) **Peptides inhibiting interleukin 1-bêta release useful as antiinflammatory agents.**

(57) Compounds of formula R-$A_1$-$A_2$-X-$A_3$ wherein R i H, a protecting group or optionally substituted benzyloxy, $A_1$ is an α-hdroxy or α-amino acid residue or a radical (α) as defined hereinafter, $A_2$ is an α-hydroxy or α-amino acid residue or $A_1$ and $A_2$ form together a pseudo-dipeptide or a dipeptide mimetics residue or a radical (aa), (bb) or (cc) as defined hereinafter, X is a residue derived from Asp wherein $A_3$ is -$CH_2$ -$X_1$-C0-$Y_1$, -$CH_2$-0-$Y_2$, -$CH_2$-S-$Y_3$ or -$CH_2$-$(C0)_m$-$Y_6$ wherein $X_1$ is 0 or S, m is 0 or 1 and $Y_1$, $Y_2$, $Y_3$ and $Y_6$ are as defined herein after, have pharmacological activity, e.g. IL-1β release inhibiting properties.

EP 0 618 223 A2

The present invention relates to peptides having pharmaceutical. utility, processes for their production, pharmaceutical compositions comprising them and their use as pharmaceuticals.

More particularly the present invention provides a compound of formula I

$$R\text{-}A_1\text{-}A_2\text{-}X\text{-}A_3 \quad (I)$$

wherein

R is hydrogen, an amino or hydroxy protecting group or optionally ring substituted benzyloxy,

$A_1$ is an α-hydroxy acid residue; an amino acid residue or a thiocarbonyl analogue thereof having each an optionally protected side chain; or a radical of formula (α)

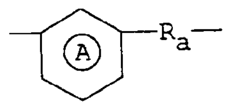

(α)

wherein ring A is optionally substituted by hydroxy or $C_{1-4}$alkoxy and $R_a$ is CO or CS

and <u>either</u>

i) $A_2$ is an α-hydroxy acid residue, -NH-CHR$_3$-CO-

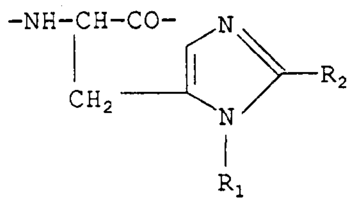

or

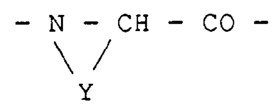

wherein

$R_1$ is $C_{1-4}$alkyl

$R_2$ is H, halogen, $C_{1-4}$alkyl, $CF_3$ or trityl,

$R_3$ is an optionally protected side chain residue of an α-amino acid, and

Y is $C_{2-5}$alkylene optionally substituted by OH, $CH_3$, $C_2H_5$, oxo, ketal or halogen,

X is a radical of formula (a) or (b)

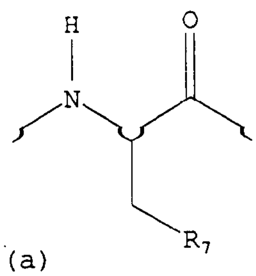

(a)

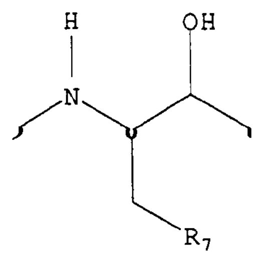

(b)

wherein $R_7$ is $-CO_2H$, -CONHOH or a bioisosteric group,

$A_3$ is $-CH_2-X_1-CO-Y_1$; $-CH_2-O-Y_2$; or $-CH_2-S-Y_3$; wherein $X_1$ is 0 or S, $Y_1$ is a cycloaliphatic residue; optionally substituted aryl; optionally ring substituted diphenylmethyl; piperidino; or optionally substituted mono-, bi- or tricyclic heteroaryl; $Y_2$ is a cycloaliphatic residue; optionally ring substituted diphenylmethyl; or optionally substituted bi- or tricyclic heteroaryl; $Y_2$ being also optionally substituted pyridyl or aryl when $A_1$ is other than an α-hydroxy acid residue and $A_2$ is other than -NH-CHR$_3$-CO-; and $Y_3$ is a cycloaliphatic residue; tri-($C_{1-4}$alkyl)methylcarbonyl, di-($C_{1-4}$alkyl)aminothiocarbonyl, 4-nitro-phenyl, 2,6-dichloro-benzoyl; 2,3,6-trichloro-4-pyridyl, a 5-membered nitrogen-containing heterocyclic radical or optionally substituted bi- or tricyclic heteroaryl; $Y_3$ being also optionally substituted pyridyl or aryl when $A_1$ is other than an α-hydroxy acid residue and $A_2$ is other than -NH-CHR$_3$-CO-;

<u>or</u>

ii) $A_2$ is a radical of formula (c)

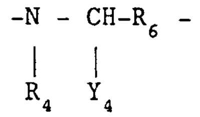 (c)

wherein

$R_4$ is H or $C_{1-4}$alkyl

$R_6$ is CO or CS

$Y_4$ is $-(CH_2)_s-N(C_{1-4}alkyl)_2$ or optionally protected $-(CH_2)_s$-NH-cyclohexyl wherein s is 1, 2, 3 or 4;

or $A_1$ and $A_2$ form together a radical of formula (aa), (bb) or (cc)

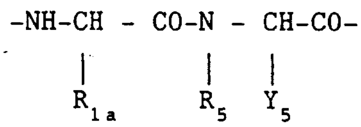

(aa)

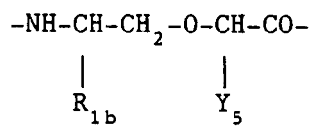

(bb)

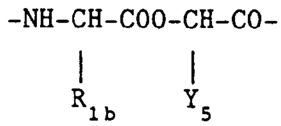

(cc)

wherein $R_{1a}$ and $R_5$ form together $C_{2-5}$alkylene or $C_{2-5}$alkenylene and $Y_5$ is an optionally protected side chain residue of an α-amino acid; or $Y_5$ has one of the significances given above for $Y_4$, or $R_5$ and $Y_5$ form together

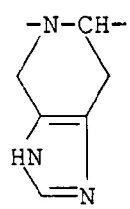

or $R_5$ is 2,3-dihydro-2-indanyl, $Y_5$ is H and $R_{1a}$ has independently one of the significance given for $R_{1b}$, and

$R_{1b}$ is an optionally protected side chain of the amino acid residue or thiocarbonyl analogue $A_1$ indicated above,

or $A_1$ and $A_2$ form together a pseudo-dipeptide or a dipeptide mimetics residue,

X is a radical of formula (a) or (b) as defined above, and

$A_3$ is $-CH_2-X_1-(CO)_m-Y_6$ wherein $X_1$ is as defined above, m is 0 or 1 and $Y_6$ is a cycloaliphatic residue; tri-($C_{1-4}$alkyl)methyl; optionally ring substituted diphenylmethyl; optionally substituted aryl; or an optionally substituted mono-, di- or tricyclic heteroaryl residue

with the provisos that

i) $Y_1$ in $A_3$ is other than optionally substituted aryl or pyridyl when $A_1$ is other than an α-hydroxy acid residue, $A_2$ is $-NH-CHR_3-CO-$ and $R_7$ in (a) or (b) is optionally esterified COOH,

ii) $Y_5$ in (aa) is in R configuration when it is the residue of an optionally protected side chain residue of an α-amino acid, $Y_6$ is optionally substituted aryl and $R_7$ is optionally esterified COOH,

and the physiologically-hydrolysable and -acceptable esters or amides thereof when $R_7$ is $-CO_2H$, in free form, in salt form or in the form of complexes.

By amino acid is meant a naturally occurring or commercially available or non natural amino acid or an optical isomer thereof. A non natural amino acid is an amino acid which is not incorporated into a protein under mRNA direction, e.g. β-Nal, a fluoro-α-amino acid such as fluoroalanine, cyclohexylalanine, trimethylsilyl-Ala,

benzimidazol-2-yl-methyl, 1H-1,2,4-triazol-3-yl-methyl, pyrazol-3-yl-methyl or indazol-3-yl-methyl.

Examples of protecting groups as R are e.g. disclosed in "Protective Groups in Organic Synthesis", T. W. Greene, J.Wiley & Sons NY (1981), 219-287, for example acyl such as formyl, acetyl, methoxysuccinyl, hydroxysuccinyl, benzoyl or phenylacetyl, each of the two latter being optionally substituted on the phenyl ring with e.g. p-methoxycarbonyl, p-methoxy or p-nitro; trityl; alkoxycarbonyl such as methoxycarbonyl, t-butyloxycarbonyl or i.-butyloxycarbonyl; allyloxycarbonyl; arylmethoxycarbonyl such as 9-fluorenylmethoxycarbonyl or benzyloxy carbonyl optionally substituted on the phenyl ring with p-methoxy, p-nitro, p-chloro or m-phenyl; aralkyl such as benzyl or phenethyl optionally ring substituted with p-methoxy, p-nitro or p-chloro, or naphthylmethyl; aralkoyl such as phenacetyl, phenylpropionyl or naphthoyl; arylsulfonyl or aralkylsulfonyl such as phenylsulfonyl or benzylsulfonyl each being optionally ring substituted with p-methyl or p-methoxy, or naphthylsulfonyl optionally ring substituted with e.g. amino or di($C_{1-4}$alkyl)amino.

Examples of hydroxy protecting groups as R are e.g. as disclosed hereinafter for aliphatic or aromatic hydroxy functionalities.

When the side chain of the natural or unnatural amino acids includes heteroatoms such as O, S or N, the heteroatoms on the side chain can optionally be protected with an O-, S- or N-protecting group.

Such protecting groups are well known in the art. N-protecting groups may be e.g. as disclosed above. O-protecting groups are e.g. described in "The Peptides", 3, 169-201. For aliphatic hydroxy functionalities, suitable O-protecting groups include the benzyl, t.-butyl and methyl groups. For aromatic hydroxy functionalities, suitable O-protecting groups include the benzyl, t.-butyl, methyl, tosyl and benzyloxycarbonyl groups. O-protecting groups for carboxy functionalities on amino acid side chains are well known ester groups, e.g. as described in "The Peptides", 3, 101-135, or amides and include the methyl, ethyl, t.-butyl and benzyl groups or amide groups such as obtained with $C_{1-4}$alkyl- amine, $C_{1-4}$alkoxy-$C_{1-4}$alkyl amine or aniline. S-protecting groups for thiol functionalities on amino acid side chains are known and described e.g. in "The Peptides", 3, 137-167. Examples include the methyl, t.-butyl, benzyl, p-methoxyphenylmethyl, ethylamino-carbonyl and benzyloxycarbonyl groups.

When $A_1$ is an amino acid residue having an optionally protected side chain, it may be an α-amino acid, e.g. Val, Leu, Ser, Ile, Gly, Ala, Phe, Pro or hydroxy-Pro, preferably Val, Leu, Ile or Ala.

When $A_1$ or $A_2$ is an α-hydroxy acid, it may be e.g. lactic acid or 2-hydroxy-3-methyl butanoic acid. Preferably only one of $A_1$ and $A_2$ is an α-hydroxy acid.

Examples of $R_3$ as an optionally protected side chain residue of an α-amino acid include a side chain residue as present in a natural α-amino acid and optionally protected, e.g. as present in Val, Leu, Ile, Ala, Thr, Ser, Cys, Met, Lys, Gly or Tyr or a radical of formula (d), (e) or (f)

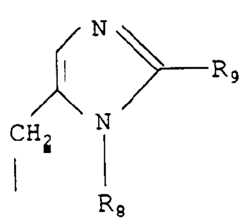

(d)

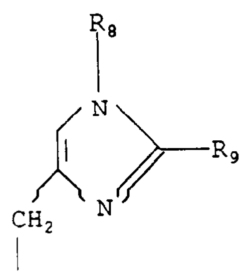

(e)

wherein each of $R_8$ and $R_9$, independently is H, halogen, $C_{1-4}$alkyl, $CF_3$ or trityl, and or

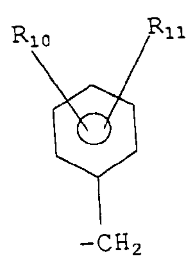

(f)

each of $R_{10}$ and $R_{11}$ independently is H, halogen, $C_{1-4}$alkyl, $C_{1-4}$alkoxy, $CF_3$, OH or phenyl.

Examples of $Y_5$ as an optionally protected side chain residue of an α-amino acid include side chain residue as present in a natural a-amino acid, e.g. Val, Leu, Ile, Ala, Thr, Ser or Lys, preferably Val, Leu, Ile or Ala, and optionally protected.

Further examples of $R_3$ include optionally protected benzimidazol-2-yl-methyl, 1H-1,2,4-triazol-3-yl-methyl, pyrazol-3-yl-methyl or indazol-3-yl-methyl.

By cycloaliphatic residue as $Y_1$, $Y_2$, $Y_3$ or $Y_6$ is meant a polycyclic cycloalkyl radical joined to the CO by a tertiary carbon atom, e.g. adamantyl.

Examples of mono-, bi- or tricyclic heteroaryl as $Y_1$ or $Y_6$ are 5- or 6-membered aromatic heterocyclic residues, optionally condensed and optionally substituted, comprising at least one heteroatom selected from N, S or O. Such heterocyclic residues may also be condensed to 1 or 2 benzene rings and/or to a further heterocyclic ring. Preferably the heteroaryl residue comprises 1, 2 or 3 heteroatoms selected from N and S. Suitable examples include optionally substituted pyridyl, indolyl, indazolyl, acridinyl, xanthenyl, pyrrolyl, benzothienyl, a 5-membered unsaturated nitrogen-containing heterocyclic radical e.g. as disclosed below or a radical of formula (g), (h), (i), (j)

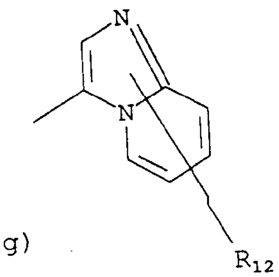

(g)

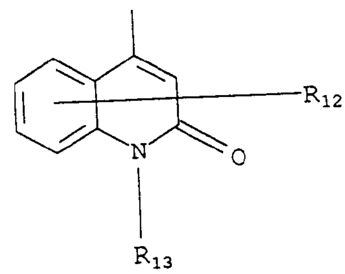

(h)

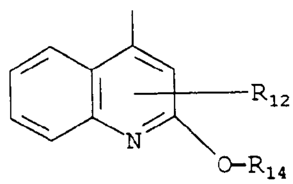

(i)

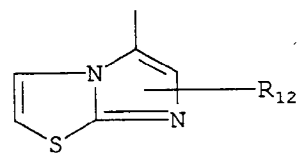

(j)

wherein $R_{12}$ is H, $C_{1-4}$alkyl, $C_{1-4}$alkoxy, $CF_3$, halogen, nitro, or cyano, and
each of $R_{13}$ and $R_{14}$ independently is H or $C_{1-4}$alkyl.

Pyridyl as $Y_1$, $Y_2$, $Y_3$ or $Y_6$ is preferably a residue attached by a carbon atom to CO or $X_1$ and optionally substituted by 1, 2 or 3 substituents selected from halogen, $CF_3$, nitro, cyano, $C_{1-4}$alkyl and $C_{1-4}$alkoxy.

Optionally substituted indolyl, indazolyl, acridinyl, xanthenyl, pyrrolyl or benzothienyl is preferably optionally protected or optionally $C_{1-4}$alkyl substituted 2- or 3-indolyl, 3-indazolyl, 9-acridinyl, 9-xanthenyl, 2-pyrrolyl, 2- or 3-benzothienyl.

Examples of bi- or tricyclic heteroaryl as $Y_2$ or $Y_3$ are optionally substituted bi- or tricyclic residues comprising at least one heteroatom selected from N, S or O and at least one unsaturated cyclic moiety, e.g. as indicated above for $Y_1$ or $Y_6$.

5-membered nitrogen-containing heterocyclic radical as $Y_3$ may comprise 2, 3 or 4 nitrogen atoms; it may be unsaturated and also optionally substituted. Suitable examples include e.g.

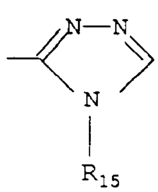

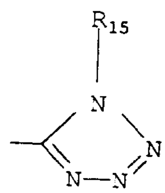

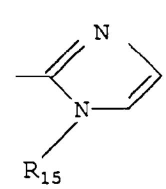

wherein $R_{15}$ is H or $C_{1-4}$alkyl.

The term "pseudo-dipeptide" as used herein refers to a dipeptide isostere in which the peptide bond between the two amino acid residues, whether natural or unnatural, is replaced with an isosteric group, e.g. C(Halogen)=CH-.

The term "dipeptide mimetics" as used herein means a chemical entity able to substitute a dipeptide in its biological properties. A "dipeptide mimetics" can have variable degrees of structural similarity with the dipeptide, mimicing e.g. only the dipeptide backbone or only the side chains of the dipeptide.

Examples of $A_1$ $A_2$ pseudo-dipeptide units include e.g.

$$- NH - CHR_{1b} - CR_{16} = CH - CHY_5 - CO -$$

wherein $R_{1b}$ and $Y_5$ are as defined above and $R_{16}$ is halogen or $C_{1-4}$alkyl.

Examples of $A_1$ $A_2$ dipeptide mimics are e.g.

- NH - $CHR_{1b}$ - $W_1$ - wherein $R_{1b}$ is as defined above and $W_1$ is 1,4-phenylene,

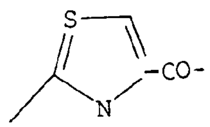

or

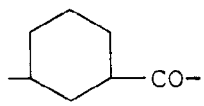

- NH - $W_2$ - wherein $W_2$ is

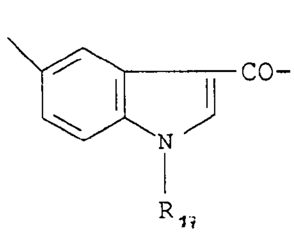

,

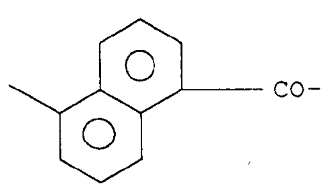

,

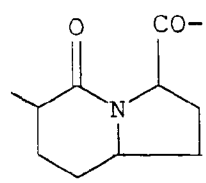

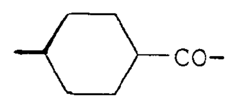

or

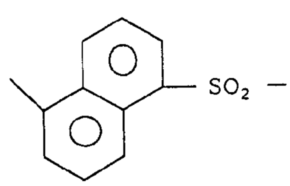

wherein $R_{17}$ is H or $C_{1-4}$alkyl,

or -O-$W_3$- wherein $W_3$ is

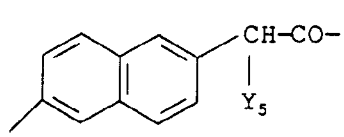

Aryl as $Y_1$, $Y_3$ or $Y_6$ means phenyl, 1- or 2-naphthyl or 9-anthracyl. Preferably 9-anthracyl is unsubstituted. Substituted phenyl as $Y_1$, $Y_3$ or $Y_6$ is phenyl bearing 1 to 5 fluoro substituents or phenyl mono-, di- or trisubstituted by substituents selected from hydroxy, $C_{1-4}$ alkyl, $C_{1-4}$alkoxy, nitro, halogen, acetyl, benzoyl, amino, $NHCH_3$, $N(C_{1-2}alkyl)_2$ cyano, $CF_3$, COOH, $CONH_2$, $C_{1-4}$alkoxycarbonyl, $NHCOC_{1-4}$alkyl, $CONHSO_2$-phenyl and $-C_6(CH_3)_4$-COOH. When $Y_1$, $Y_3$ or $Y_6$ is naphthyl, it may be unsubstituted naphthyl or 1-naphthyl substituted in position 2 by $C_{1-4}$alkyl, $C_{1-4}$alkoxy or $CF_3$.

Halogen means fluorine or chlorine.

By bioisosteric group as $R_7$ is meant a group replacing one part of a biologically active molecule without altering its essential biological features. Suitable examples of a bioisosteric group for carboxy include e.g. a tetrazolyl group.

Radical of formula (a) comprises one asymetric carbon and radical of formula (b) comprises two asymetric carbons. Accordingly these radicals lead to optical isomerism. It will be understood that the present invention includes all individual isomeric forms and diastereoisomers as well as mixtures, e.g. racemates unless otherwise stated.

By the term "physiologically-hydrolysable and -acceptable esters or amides" are meant esters and amides which are hydrolysable under physiological conditions to yield alcohols or amines which are themselves physiologically acceptable, i.e. which are non-toxic at the desired dosage levels.

Such esters or amides are obtained by esterification or amidation, respectively, of a compound of formula I wherein $R_7$ is a carboxy group. Such esters include esters with an aliphatic or alicyclic alcohol or polyol having 1 to 12 carbon atoms. Such amides include amides with aliphatic amines, e.g. $C_{1-4}$alkyl amine, $C_{1-4}$alkoxy-$C_{1-4}$alkyl amine such as β-methoxy-ethyl amine, or aniline.

The compounds of formula I may exist e.g. in free form, acid addition salt form or in the form of complexes thereof. Acid addition salts may be formed with e.g. organic acids, polymeric acids and inorganic acids. Such acid addition salt forms include e.g. the hydrochlorides and acetates. Salt forms may also include those obtainable with the carboxylic group present in compounds of formula I, e.g. alkali metal salts such as sodium or potassium, or substituted or unsubstituted ammonium salts. Complexes are e.g. formed from compounds of formula I on addition of inorsubstances, e.g. inorganic salts or hydroxides such as Ca- and Zn-salts, and/or an addition of polymeric organic substances.

All α-amino acid residues present may have the D or L configuration, except otherwise stated, e.g. as in proviso (ii).

In the compounds of formula I, the following significances are preferred either individually or in any combination or sub-combination:

1. R is an amino protecting group or benzyloxy, preferably an amino protecting group e.g. benzyloxycarbonyl, naphthoyl naphthylsulfonyl optionally ring-substituted by di($C_{1-4}$alkyl)-amino. R is preferably benzyloxy when $A_1$ is a radical of formula (α).
2. $A_1$ is Val, D-Val, Phe, Leu, Ala, Ile, trimethylsilyl-Ala or an α-hydroxy acid residue.
3. X is a radical of formula (a).
4. Monocyclic heteroaryl is optionally substituted pyridyl or 5-membered unsaturated nitrogen-containing heterocyclic radical.
5. $A_2$ is

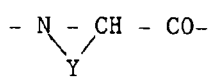 ;

$A_3$ is $-CH_2-X_1-CO-Y_1$, $-CH_2-O-Y_2$ or $-CH_2-S-Y_3$.

6. $A_1$ is an α-hydroxy acid residue; $A_2$ is $-NH-CHR_3-CO-$; $A_3$ is $-CH_2-X_1-CO-Y_1$, $-CH_2-O-Y_2$ or $-CH_2-S-Y_3$.
7. $Y_1$ is substituted aryl, optionally substituted heteroaryl or a cycloaliphatic residue.
8. $A_2$ is $-NH-CHR_3-CO-$; $A_3$ is $-CH_2-X_1-CO-Y_1$ wherein $Y_1$ is a cycloaliphatic residue or optionally substituted bi- or tricyclic heteroaryl, or $-CH_2-S-Y_3$ wherein $Y_3$ is a cycloaliphatic residue or a 5-membered nitrogen-containing heterocyclic radical.
9. $A_2$ is an α-hydroxy acid residue; $A_3$ is $-CH_2-X_1-CO-Y_1$, $-CH_2-O-Y_2$ or $-CH_2-S-Y_3$. $Y_1$, $Y_2$ or $Y_3$ is more preferably a cycloaliphatic residue or optionally substituted aryl or pyridyl.
10. In radical (c) $R_4$ is H.
11. $R_6$ is CO.
12. $A_1$ and $A_2$ form together a radical of formula (aa) wherein $R1_a$ and $R_5$ form together $C_{2-5}$alkylene, $-CHY_5-CO-$ has the D configuration and $A_3$ is $-CH_2-X_1-(CO)_m-Y_6$ wherein $Y_6$ is optionally substituted aryl or pyridyl.
13. $A_1$ and $A_2$ form together a radical of formula (aa) wherein $R_{1a}$ and $R_5$ form together $C_{2-5}$alkylene, and $A_3$ is $-CH_2-X_1-(CO)_m-Y_6$ wherein $Y_6$ is other than optionally substituted aryl or pyridyl.

14. $A_1$ and $A_2$ form together a pseudo-dipeptide residue.

15. $A_1$ and $A_2$ form together a dipeptide mimetics residue; $Y_6$ is optionally substituted aryl or optionally substituted mono-, di- or tricyclic heteroaryl.

16. Preferred dipeptide mimetics residues are $-NH-CHR_{1b}-W_1-$ wherein $W_1$ is

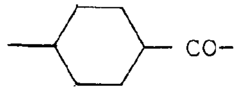 ;

$-NH-W_2$ wherein $W_2$ is

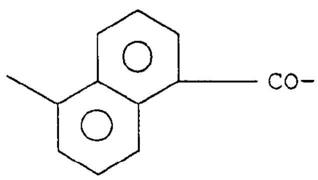

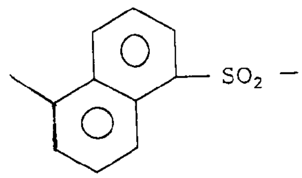

or 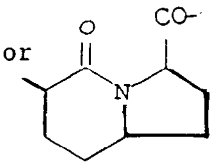 ; $-O-W_3$

17. $R_7$ is $-CO_2H$ or an ester thereof, preferably $-CO_2H$ or ($C_{1-12}$alkoxy)carbonyl.

The present invention also provides a process for the production of a compound of formula I, which process comprises:

a) removing at least one protecting group from a compound of formula I in protected form or adding a protecting group R at the terminal group of a compound of formula I; or

b) converting one compound of formula I into another compound of formula I; or

c) coupling together by an amide bond two peptide fragments, each of which contains at least one amino acid in protected or unprotected form and one peptide fragment containing a radical of formula (a) or (b) as defined above, the peptide fragments being such that a protected or unprotected peptide having the sequence according to formula I above is obtained and, if necessary, removing the protecting group or groups from a compound of formula I in protected form; or

d) reacting a compound of formula II

$$R-A_1-A_2-X-CH_2-Z_a \qquad (II)$$

wherein R, $A_1$, $A_2$ and X are as defined above, and $Z_a$ is a leaving group, with a compound of formula $HX_1(CO)_m-Y_7$ wherein $X_1$ and m are as defined above and $Y_7$ is $Y_1$, $Y_2$, $Y_3$ or $Y_6$ or a functional derivative thereof when m is 1;

e) for the production of a compound of formula I wherein X is a radical of formula (a) or (b) monoalkylating or monoacylating a compound of formula III

$$R-A_1-A_2-NH-CH(CH_2-R_{18})-CHOH-CH_2OH \qquad (III)$$

wherein R, $A_1$ and $A_2$ are as defined above and $R_{18}$ is an ester group, with a compound $HX_1-(CO)_m-Y_7$ wherein m, $X_1$ and $Y_7$ are as defined above, or a functional derivative thereof, when m is 1.

and recovering a compound of formula I thus obtained in free or salt form or in the form of a complex.

Processes (a) to (e) above may be carried out in accordance with standard techniques known in the art.

The removal of a protecting group in process step (a) may also include the removal of R on the terminal group of a compound of formula I. For example, when R is benzyloxy carbonyl, this group may be removed by hydrogenation in the presence of a catalyst, e.g. Pd.

In accordance with process step (b) for example, for the production of a compound of formula I wherein X comprises a carboxy group, a compound of formula I wherein X comprises an esterified or amidated carboxy

group may be hydrolysed. Such hydrolysis may be effected by treatment with an appropriate alkali or by acid hydrolysis, for example in the presence of trifluoroacetic acid.

Furthermore, in accordance with process step (b), for the production of a compound of formula I wherein X comprises an esterified or amidated carboxy group, a compound of formula I wherein X comprises a carboxy group or an esterified caboxy group may be (trans) esterified or amidated. Such ester formation or amidation may be carried out using any of the techniques known in the art, for example converting the carboxy group in a functional reactive group, e.g. a corresponding acid halide or anhydride.

In accordance with a further embodiment of process step (b), for the production of a compound of formula I wherein X is a radical of formula (a), a compound of formula I wherein X is a radical of formula (b) may be oxidized. This process may be carried out in analogy to the known techniques used for the oxidation of an alcohol, e.g. according to Jones, Swern or Pfitzner-Moffatt oxidation procedures.

Process step (c) may be carried out by the techniques known in the art of peptide chemistry.

Functional derivatives of $HX_1$-$(CO)_m$-$Y_6$ when m is 1 include e.g. acid halides, for example acid chloride, anhydride, etc. $Z_a$ may be e.g. halogen.

Process step (e) may also be carried out according to an esterification procedure, e.g. according to the Mitsunobu procedure.

Where desired, in these reactions, protecting groups may be used for functional groups which do not participate in the reaction. These may be e.g. amino protecting groups, carboxy protecting groups, acetal groups etc. When the desired reaction is complete, the protecting groups may then be removed.

Each of the above processes may be carried out using starting materials in the form of one or other of the individual optical isomers or in the form of mixtures [relating to the asymetric carbons present in radicals of formula (a) or (b) as X or in such a radical precursor]. Conveniently the starting materials are used as S- or R-enantiomers to produce a compound of formula I wherein the asymetric carbon in radicals of formulae (a) or (b) has the S or R configuration, respectively.

The starting materials used in process steps (d) or (e) may be prepared in analogy with process step (c). Insofar as the production of the starting materials is not particularly described, the compounds are known or may be prepared analogously to methods known and practiced in the art.

The following examples are illustrative of the invention. All temperatures are in °C.

The following abbreviations are used:

| | |
|---|---|
| THF | = tetrahydrofuran |
| TFA | = trifluoroacetic acid |
| MeOH | = methanol |
| EtOAc | = ethyl acetate |
| DCC | = dicyclohexylcarbodiimide |
| HOBT | = hydroxybenzotriazole |
| Z | = benzyloxycarbonyl |
| Pip | = pipecoline |
| BOC | = t.-butoxycarbonyl |
| Fmoc | = 9-fluorenylmethoxycarbonyl |
| EDCI | = N-ethyl-N'-dimethylaminopropyl-carbodiimide hydrochloride |
| DMAP | = 4-dimethylaminopyridin |
| DAST | = diethylamino sulphur trifluoride |
| t.Bu | = tert.-butyl |

## EXAMPLE 1:

**(3R)-3-[3-(3-benzyloxybenzoyl)aminobenzoyl]-amino-(4S)-4-hydroxy-5(1-adamantoyloxy)pentanoic acid ethyl ester**

3-(3-benzyloxybenzoyl)aminobenzoic acid (0.44 g, 1.2 mmol) is dissolved in DMF (15 ml), HOBt.$H_2O$ (0.2 g, 1.2 mmol) and DCC (0.53 g, 2.55 mmol) added, followed by (3R)-amino-(4S)-4-hydroxy-5(1-adamantoyloxy)pentanoic acid ethyl ester (0.4 g, 1.2 mmol) in DMF (4 ml). The reaction mixture is stirred over night, filtered and DMF evaporated. The residue is dissolved in EtOAc, adjusted to alkaline pH by adding $NH_4OH$ conc. and chromatographed ($SiO_2$, aceton/hexane 15/85), yielding the title compound as a colorless foam.

**EXAMPLE 2:**

**Z-valyl-(π-ethyl)histidinyl]-(3RS)-3-amino-5(2,6-dichlorobenzoyloxy)-4-oxo-pentanoic acid ethyl ester**

[Z-valyl-(t-BOC)histidinyl]-(3RS)-3-amino-5(2,6-dichlorobenzoyloxy)-4-oxo-pentanoic acid ethyl ester (500 mg, 0.61 mmol) is dissolved in $CH_2Cl_2$ (10 ml). Ethyltriflate (0.24 ml, 1.83 mmol) is added at -78°. The reaction mixture is brought to room temperature within 6 hours and then stirred for another 20 hours at room temperature. Water is added and the mixture extracted with EtOAc. The combined organic phases are washed with saturated $NaHCO_3$, then with a NaCl solution, dried over $Na_2SO_4$ and evaporated to dryness. The residue is purified by chromatography ($SiO_2$, EtOAc/EtOH/$NH_4OH$ 97/2/1), yielding the title compound as a colorless foam.

The compound used as starting material may be produced as follows:

(Z-valyl-histidinyl)-(3RS)-3-amino-5(2,6-dichlorobenzoyloxy)-4-oxo-pentanoic acid ethyl ester (0.5 g, 0.7 mmol) is dissolved in THF (10 ml) and added to $(BOC)_2$ 0 (0.26 g, 1.2 mmol) in THF (10 ml and stirred for 48 hours at room temperature. The solvent is evaporated and the product used without further purification.

**EXAMPLE 3:**

**(3S)-3[Z-valyl-(4S)-4-fluoroprolinyl]amino-4-oxo-5(2,6-dichlorobenzoyloxy)pentanoic acid tert.butyl ester**

(3S)-3[(Z-valyl-(4S)-4-fluoro]prolinyl)]amino-(4RS)-4-hydroxy-5(2,6-di-chlorobenzoyloxy)pentanoic acid tert.butyl ester (0.84 g, 1.16 mmol) is dissolved in acetone (40 ml). Jones reagent (4 ml, 9.2 mmol) is added in 8 portions at 5 min. intervals. When the addition is complete, the reaction mixture is stirred for an additional 2.75 hours at room temperature, adjusted to alkaline pH by adding 25 % $NH_4OH$ and filtered over $SiO_2$. Evaporation of the solvents give 0.7 g of crude product, which is purified by chromatography ($SiO_2$, acetone/hexan 25/75), yielding the title compound as a white powder.

**EXAMPLE 4:**

**(3S)-3[Z-valyl-(4S)-4-fluoroprolinyl]amino-(4RS)-4-hydroxy-5-(2,6-di-chlorobenzoyloxy)pentanoic acid tert.butyl ester**

Z-valyl-(4S)-4-fluoroproline (0.5 g, 1.36 mmol), DCC (0.56 g, 2.7 mmol) and HOBt.$H_2O$ (0.21 g, 1.36 mmol) are dissolved in THF (10 ml) and combined with (3S)-amino-(4RS)-4-hydroxy-5-(2,6-dichlorobenzoyloxy)pentanoic acid tert.butyl ester in THF (10 ml). The reaction mixture is stirred at room temperature for 20 hrs. and filtered. The filtrate is evaporated, taken up in EtOAc, made alkaline with $NH_4OH$, filtered over Hyflo and evaporated again. The residue is chromatographed over $SiO_2$ (acetone/hexane 3/7) to yield the desired product as a white foam.

By following the procedures of Examples 1-4 above or in analogy with Examples 1-3 or 11-13 of WO 93/09135, the compounds of formula

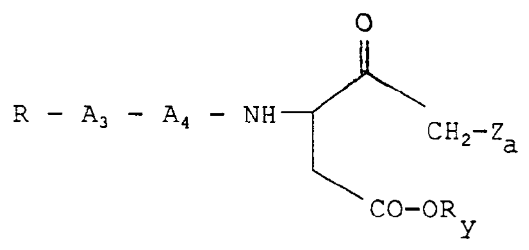

wherein R, $A_3$, $A_4$, $R_x$, $R_y$ and $Z_a$ are as defined in the Table below, may be produced.

| Ex. | R | $A_3$ | $A_4$ | Ry | Za | Characterization | |
|---|---|---|---|---|---|---|---|
| 5 | Z | Val | Ala | Et | 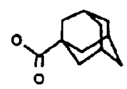 | 3R(1) | 642(2) |
| 6 | Z | Val | Ala | H | id. | 3R | 614 |
| 7 | Z | Val | Ala | t.Bu | id. | 3R | 670 |
| 8 | Z | Val | Ala | Et | id. | 3R,S(1) | 642 |
| 9 | Z | Val | 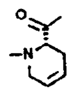 | Et | id. | 3R | 680 |
| 10 | Z | Val | 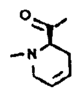 | Et | id. | 3R | 680 |
| 11 | Z | Val | Ala | H | 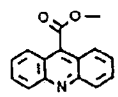 | | 657 |
| 12 | Z | Val | Ala | t.Bu | id. | | 713 |
| 13 | Z | Val | Ala | H | 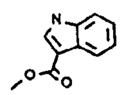 | | 595 |
| 14 | Z | Val | Ala | t.Bu | id. | | 651 |
| 15 | Z | Val | Ala | H | 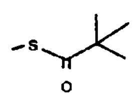 | | 552 |
| 16 | Z | Val | Ala | t.Bu | id. | | 608 |
| 17 | Z | Val | Ala | Et | id. | 3R | 580 |

| Ex. | R | $A_3$ | $A_4$ | Ry | $Z_a$ | Characterization |
|---|---|---|---|---|---|---|
| 18 | Z | Val | D-Ala | Et | 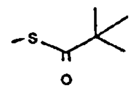 3R | 580 |
| 19 | Z | Val | Ala | H | S-CS-N(Et)$_2$ | 583 |
| 20 | Z | Val | Ala | t.Bu | id. | 639 |
| 21 | Z | Val | Ala | Et | 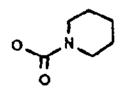 3R | 591 |
| 22 | Z | Val | Ala | H | 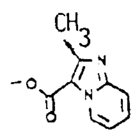 | 610 |
| 23 | Z | Val | Ala | t.Bu | id. | 666 |
| 24 | Z | Val | Ala | H | 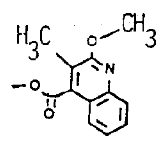 | 651 |
| 25 | Z | Val | Ala | Et | id. | 679 |
| 26 | Z | Val | Ala | H | 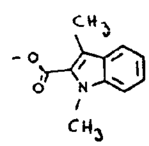 | 623 |
| 27 | Z | Val | Ala | t.Bu | id. | 679 |
| 28 | Z | Val | Ala | Et | id. | 651 |
| 29 | Z | Val | Ala | H | 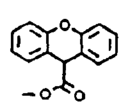 | 660 |

| Ex. | R | $A_3$ | $A_4$ | $R_y$ | $Z_a$ | Characterization |
|---|---|---|---|---|---|---|
| 30 | Z | Val | Ala | H | 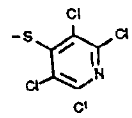 | 683 |
| 31 | Z | Val | Ala | H | 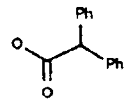 | . 646 |
| 32 | Z | Val | Ala | H | 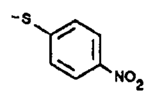 | 589 |
| 33 | Z | Val | Ala | t.Bu | id. | 645 |
| 34 | Z | Val | Ala | H | 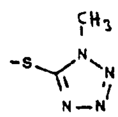 | 550 |
| 35 | Z | Val | Ala | H | 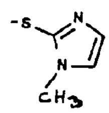 | 549 |
| 36 | Z | Val | Ala | t.Bu | id. | 605 |
| 37 | Z | Val | Ala | H | 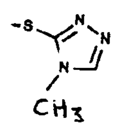 | 548 |
| 38 | Z | Val | Ala | t.Bu | id. | 604 |
| 39 | Z | Val | Ala | Et | 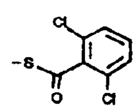 3R | 669 |

| Ex. | R | $A_3$ | $A_4$ | $R_y$ | $Z_a$ | Characterization | |
|---|---|---|---|---|---|---|---|
| 40 | Z | Val | Lys ($N^\varepsilon$-acetyl $N^\varepsilon$-cyclohexyl) | Et | 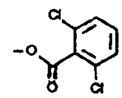 | | 834 |
| 41 | 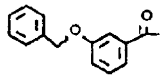 | | 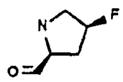 | t.Bu | id. | 3S | 702 |
| 42 | id. | id. | id. | H | id. | 3S | 646 |
| 43 | Z | 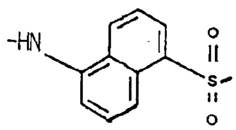 | | H | id. | 3S | 660 |
| 44 | Z | id. | id. | t.Bu | id. | 3S | 716 |
| 45 | Z | id. | id. | Et | id. | 3S | 688 |
| 46 | Z | 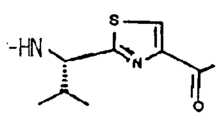 | | Et | id. | 3R | 665 |
| 47 | Z | id. | id. | H | id. | 3R,S | 637 |
| 48 | Z | 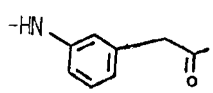 | | Et | id. | 3R | 3.3°(3) |

| Ex. | R | $A_3$ | $A_4$ | $R_y$ | $Z_a$ | Characterization | |
|---|---|---|---|---|---|---|---|
| 49 | Z | 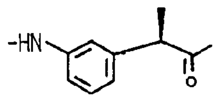 | | Et | 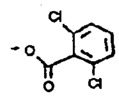 | 3R | 4.6°(3) |
| 50 | Z | id. | id. | H | id. | 3S | -0.5°(3) |
| 51 | Z | id. | id. | t.Bu | id. | 3R | -0.5°(3) |
| 52 | Z | id. | id. | H | id. | 3R | -0.2°(3) |
| 53 | Z | 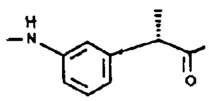 | | Et | id. | 3R | 6.0°(3) |
| 54 | Z | id. | id. | H | id. | 3R | 601.5 |
| 55 | Z | id. | id. | t.Bu | id. | 3R | 0.2°(3) |
| 56 | Z | 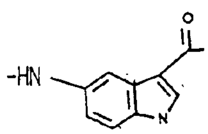 | | H | id. | 3S | 613 |
| 57 | Z | id. | | Et | id. | 3R | 641 |
| 58 | Z | 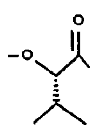 | Ala | H | id. | 3R,S | -23°(3) |
| 59 | Z | id. | Ala | Et | id. | 3R | 16.6°(3) |
| 60 | Z | id. | Ala | t.Bu | id. | 3R,S | |
| 61 | Z | Val | 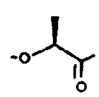 | H | id. | 3R,S | -11.1°(3) |
| 62 | Z | Val | id. | t.Bu | id. | 3R,S | -6.9°(3) |
| 63 | Z | Val | id. | Et | id. | 3R | 23.4°(3) |

| Ex. | R | $A_3$ | $A_4$ | $R_y$ | $Z_a$ | | Characterization |
|---|---|---|---|---|---|---|---|
| 64 | Z | Val | id. | Et | 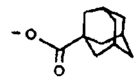 | 3R | 19.9°(3) |
| 65 | Z | Ala | Pro | H | 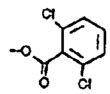 | | 623 |
| 66 | Z | Ala | Pro | t.Bu | id. | | 679 |
| 67 | Z | Val | 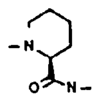 | H | id. | | 665 |
| 68 | Z | Val | id. | t.Bu | id. | | 721 |
| 69 | Z | Val | 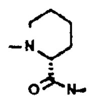 | H | id. | | 665 |
| 70 | Z | Val | id. | t.Bu | id. | | 721 |
| 71 | Z | Val | Spinacin | H | id. | | 703 |
| 72 | Z | Val | id. | t.Bu | id. | | 759 |
| 73 | Z | Val | Pro | H | id. | | 651 |
| 74 | Z | Val | Pro | Et | id. | | 679 |
| 75 | Z | Val | Pro | Et | id. | 3R | 679 |
| 76 | 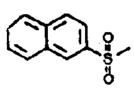 | Val | Ala | Et | 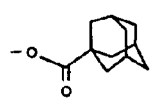 | 3R | 698 |
| 77 | 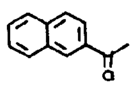 | Val | Ala | Et | id. | 3R | 662 |

| Ex. | R | $A_3$ | $A_4$ | $R_y$ | $Z_a$ | Characterization | |
|---|---|---|---|---|---|---|---|
| 78 | Z | 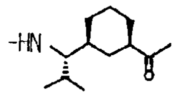 | | Et | 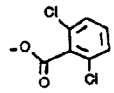 | | 664 |
| 79 | Z | 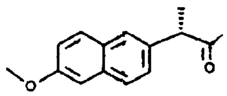 | | Et | id. | 3R | 53.4°(4) |
| 80 | Z | id. | id. | Et | id. | 3S | -30°(4) |
| 81 | Z | id. | id. | H | id. | 3R | 44.4°(4) |
| 82 | Z | id. | id. | H | id. | 3S | -19.5°(4) |
| 83 | Z | 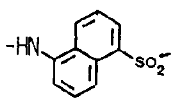 | | H | id. | 3S | 660 |
| 84 | Z | id. | id. | t.Bu | id. | 3S | 716 |
| 85 | Z | id. | id. | Et | id. | 3R | 688 |
| 86 | Z | 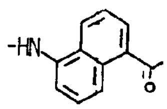 | | H | id. | 3R,S | 624 |
| 87 | Z | 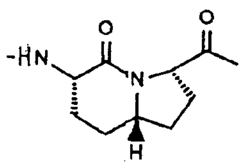 | | H | id. | 3S | 635 |
| 88 | Z | Val | 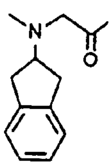 | H | id. | 3R,S | 727 |
| 89 | Z | Val | id. | t.Bu | id. | 3R,S | 783 |

| Ex. | R | $A_3$ | $A_4$ | $R_y$ | $Z_a$ | Characterization | |
|---|---|---|---|---|---|---|---|
| 90 | Z | 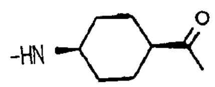 | | H | 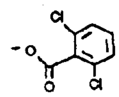 | 3R,S | 580 |
| 91 | Z | id. | id. | t.Bu | id. | 3R,S | 636 |
| 92 | Z | 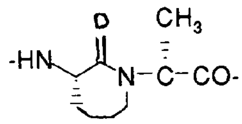 | | H | id. | 3S | 637 |
| 93 | Z | id. | id. | t.Bu | id. | 3S | 697 |
| 94 | Z | id. | id. | Et | id. | 3S | 665 |
| 95 | Z | -NH-CH-CCl=$CCH_3$-CO-<br>$CH(CH_3)_2$ | | H | id. | | 6°(4) |
| 96 | Z | $CH_3$<br>-NH-CH-CCl=C-CO-<br>$CH(CH_3)_2$ | | H | id. | | -21°(4) |

(1): configuration

(2): $MH^+$ (MS m/z)

(3): $[\alpha]_D^{20}$ in $CHCl_3$

(4): $[\alpha]_D^{20}$ in methanol

Z protected 2-(1-(S)-aminoisobutyl)thiazole-4-carboxylic acid is used in the preparation of compound of Example 46 and is prepared according to the method described by T.Shiori et al. in J.Org.Chem. 1987, 52, 1252 from Z-valinal and cysteine methyl ester.

Z protected racemic cis-3-(1-aminoisobutyl)cyclohexane-1-carboxylic acid is used in the preparation of compound of Example 78 and is prepared as a racemate by a multiple synthesis: 3-oxabicyclo[3.3.1]nonane-2,4-dione (T. Yamazaki et al. Chem. Pharm. Bull. 1987, 35(8), 3453) is reacted with Li-salt of isobutyric acid t.-butyl ester. The resulting acid is converted into its methyl ester, the t.butyl ester deesterified with TFA and decarboxylated at 145° to provide cis-3-(1-oxo-2-methylpropyl)-1-cyclohexane carboxylic acid methyl ester. The latter keto-ester is converted into its oxime and hydrogenated over $PtO_2$ in acetic acid to the corresponding amino ester which is cyclized upon heating to 50° to give two lactams (2:1) separated by fractional crystallization. The major component (3-azabicyclo[3.3.1]nonane-3-(endo)isopropyl-1-one: m.p.133-134.5°, crystallized from ether/hexane) is hydrolized to the corresponding amino acid by refluxing in 35% HCl for four days and protected with Z.

Z protected (3S,6S,S9)-6amino-5-oxo-indolizidin-3-carboxylic is used in Example 87 and is prepared by reacting 2,2-bis(2-iodoethyl)-1,3-dioxolane with 2 equivalents of (2R)-(-)2,5-dihydro-3,6-dimethoxy-2-isopropylpyrazine accroding to the "bislactimether method" developed by Schoellkopf (U. Schoellkopf, Pure & Appl. Chem. 1983,55,1799) to yield after hydrolysis of the intermediate ketal and reductive amination (Pd/C, ethanol, 1 atm $H_2$)followed by spontaneous cyclization the desired indolizidine derivative. The free amino group is Z-protected and the ester hydrolized to give the desired indolizidine derivative.

Z protected (2R,3Z,5S)-5-amino-4-chloro-2,6-dimethyl-3-heptenoic acid used in the preparation of compound of example 96 may be prepared as follows: the allylic alcohol resulting of a syn-aldol-condensation of N-propionyl-10,2-sultam with the (E/Z)-2-chloro-4-methyl-2-pentenal as described by W. Oppolzer [JACS 112, 2767 (1990)] is thermally rearranged as its trichloroacetimidate [L.E. Overman, JACS, 98, 2901 (1976)]. This intermediate is hydrolyzed to the free amino acid and then protected.

**EXAMPLE 97**:

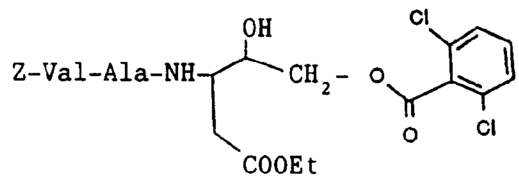

$MH^+$ : 644

The compounds of formula I their physiologically-hydrolysable and -acceptable esters and amides and their pharmaceutically acceptable salts (hereinafter referred to as compounds of the invention) exhibit pharmaceutical activity and are, therefore, useful as pharmaceuticals.

In particular, the compounds of the invention inhibit IL-1β secretion as indicated in the following in vitro test using THP-1 cells and in vivo test methods:

a) 900 μl THP-1 cells ($0.5 \times 10^6$ cells) together with 100 U γ-interferon/0.9 ml RPMI 1640 medium (containing 2 mM L-glutamine and 5 % heat-inactivated foetal calf serum) are pipetted into 24 well culture plates. 100 μl of the compound to be tested are then added. After 3 hours at 37 ° C in 5 % $CO_2$/95 % air, 10 μl lipopolysaccharide 500 μg/ml is added and the incubation continued for a further 40 hours. Appropriate controls (with and without stimulus, solvent) ar also included. The media are then removed and clarified by centrifugation at 1000 g for 10 min. 1.0 ml digitonin 0.01 % is added to the wells to lyse the cells which are loosened by scraping with a rubber policeman and left at 4 ° C for 10 min. Lactate dehydrogenase measurements are then performed immediately and the samples stored at - 20 ° C until the other determinations can be made. The assays are: IL-1β (medium and lysate), IL-6 (medium), TNF-α (medium), PGE2 (medium and lysate), lactate dehydrogenase (LDH) and DNA (lysates). IL-1β, IL-6 and TNF-α assays are determined using commercially available ELISA kits (Cistron), $PGE_2$ is measured using a standard RIA and DNA fluorimetrically using DAPI.

In this test, the compounds of the invention selectively inhibit IL-1β release in concentrations from about 0.01 to 100 μM. In contrast IL-6, TNF-α, PGE2 and DNA levels remain substantially unaffected, and the compounds are non-toxic, since LDH release is unchanged.

b) **LPS-Fever**

A LPS-suspension (Sigma, No. L-5886; 100μg/5ml glucose solution/kg s.c.) is injected in male Tuttlingen SD rats (150-160g). 2 hours later the body temperature is measured using a thermistor rectal probe connected to an ELLAB telethermometer. After 4 hours the test compound is administered p.o. 2 hours later (6 hrs after LPS administration) the temperature is measured again. The temperature increment shown by the untreated controls is taken as 100% and that in the treated group is expressed as a percentage of this value. The $ED_{50}$ is the dose causing a 50% inhibition of the temperature increase determined in the control rats. In this test compounds of the invention inhibit the LPS-induced temperature increase when administered at a dosage in the range of from 0.001 to 0.1 mg/kg p.o.

Compounds of the invention are therefore indicated for use in the treatment of disorders with an aetiology associated with or comprising excessive IL-1β release, e.g. in a wide variety of inflammatory states and diseases, for example tissus calcium depletion, degenerative processes in bone and cartilage, e.g. rheumatoid arthritis and osteoarthritis, inflammatory bowel disease, irritable bowel disease, septic shock, psoriasis, asthma, adult respiratory distress syndrome, diabetes type I, allergic skin disorders, osteoporosis of various genesis including e.g. climacteric or post-menopausal osteoporosis as well as osteoporosis consequential to old age, immobilization or trauma, arteriosclerosis, Alzheimer disease, acute or chronic myelogenous leukemia or a variety of autoimmune disease states.

For the above indications the required dosage will of course vary depending upon, for example, the compound of the invention employed, the mode of administration, the particular condition to be treated and the effect desired. An indicated daily dosage is of the order of from about 0.1 mg to about 1 g/day, conveniently administered once, in divided dosages 2 to 4 x/day, or in sustained release form.

In accordance with the foregoing the present invention also provides:

a) A compound of formula I, a physiologically-hydrolysable and -acceptable ester or amide thereof or a pharmaceutically acceptable salt thereof, for use as a pharmaceutical, for example in the treatment of above indicated disorders, e.g. for use as an anti-inflammatory agent.

The compounds of the invention may be administered by any conventional route, in particular nasally, enterally, e.g. orally, e.g. in the form of tablets or capsules, or parenterally e.g. in the form of injectable solutions or suspensions or in a suppository form. Unit dosage forms contain, for example from about 25 μg to 500 mg of a compound of the invention.

The compounds of the invention may be administered in free form or in pharmaceutically acceptable salt form. Such salts may be prepared in conventional manner and exhibit the same order of activity as the free compounds.

Furthermore the present invention also provides:

b) A pharmaceutical composition comprising a compound of formula I, a physiologically-hydrolysable and -acceptable ester or amide thereof or a pharmaceutically acceptable salt thereof, as hereinbefore defined, together with a pharmaceutically acceptable diluent or carrier therefor. Such compositions may be manufactured in conventional manner.

Compounds of the invention have an interesting pharmacological profile in view of their in vitro and in vivo activity. Compounds of formula I wherein $A_2$ is optionally substituted proline, or $A_1$ and $A_2$ form together a radical of formula (aa) or a dipeptide mimetics residue are particularly preferred.

## Claims

1. A compound of formula I

$$R\text{-}A_1\text{-}A_2\text{-}X\text{-}A_3 \qquad (I)$$

wherein

R is hydrogen, an amino or hydroxy protecting group or optionally ring substituted benzyloxy,

$A_1$ is an α-hydroxy acid residue; an amino acid residue or a thiocarbonyl analogue thereof having each an optionally protected side chain; or a radical of formula (α)

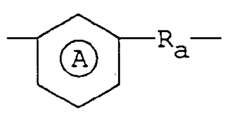

(α)

wherein ring A is optionally substituted by hydroxy or $C_{1-4}$alkoxy and $R_a$ is CO or CS

and <u>either</u>

i) $A_2$ is an α-hydroxy acid residue, -NH-CHR$_3$-CO-

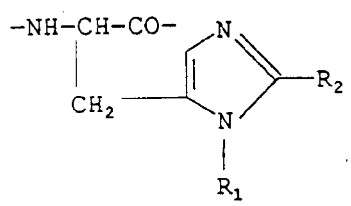

or

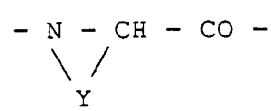

wherein

| | |
|---|---|
| $R_1$ | is $C_{1-4}$alkyl |
| $R_2$ | is H, halogen, $C_{1-4}$alkyl, $CF_3$ or trityl, |
| $R_3$ | is an optionally protected side chain residue of an α-amino acid, and |
| Y | is $C_{2-5}$alkylene optionally substituted by OH, $CH_3$, $C_2H_5$, oxo, ketal or halogen, |
| X | is a radical of formula (a) or (b) |

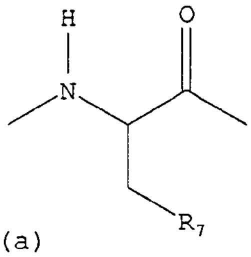

(a)

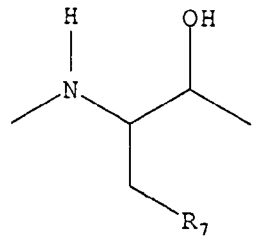

(b)

wherein $R_7$ is $-CO_2H$, -CONHOH or a bioisosteric group,

$A_3$ is $-CH_2-X_1-CO-Y_1$; $-CH_2-O-Y_2$; or $-CH_2-S-Y_3$; wherein $X_1$ is O or S, $Y_1$ is a cycloaliphatic residue; optionally substituted aryl; optionally ring substituted diphenylmethyl; piperidino; or optionally substituted mono-, bi- or tricyclic heteroaryl; $Y_2$ is a cycloaliphatic residue; optionally ring substituted diphenylmethyl; or optionally substituted bi- or tricyclic heteroaryl; $Y_2$ being also optionally substituted pyridyl or aryl when $A_1$ is other than an α-hydroxy acid residue and $A_2$ is other than $-NH-CHR_3-CO-$; and $Y_3$ is a cycloaliphatic residue; tri-($C_{1-4}$alkyl)methylcarbonyl, di-($C_{1-4}$alkyl)aminothiocarbonyl, 4-nitro-phenyl, 2,6-dichloro-benzoyl; 2,3,6-trichloro-4-pyridyl, a 5-membered nitrogen-containing heterocyclic radical or optionally substituted bi- or tricyclic heteroaryl; $Y_3$ being also optionally substituted pyridyl or aryl when $A_1$ is other than an α-hydroxy acid residue and $A_2$ is other than $-NH-CHR_3-CO-$;

<u>or</u>

ii) $A_2$ is a radical of formula (c)

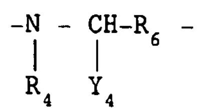

(c)

wherein

$R_4$ is H or $C_{1-4}$alkyl

$R_6$ is CO or CS

$Y_4$ is $-(CH_2)_s-N(C_{1-4}alkyl)_2$ or optionally protected $-(CH_2)_s-NH$-cyclohexyl wherein s is 1, 2, 3 or 4;

or $A_1$ and $A_2$ form together a radical of formula (aa), (bb) or (cc)

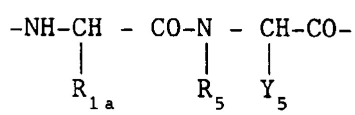

(aa)

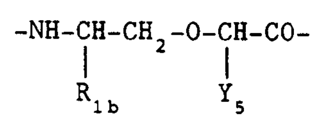

(bb)

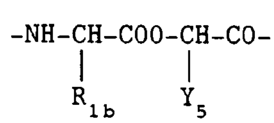

(cc)

wherein $R_{1a}$ and $R_5$ form together $C_{2-5}$alkylene or $C_{2-5}$alkenylene and $Y_5$ is an optionally protected side chain residue of an α-amino acid; or $Y_5$ has one of the significances given above for $Y_4$,

or $R_5$ and $Y_5$ form together

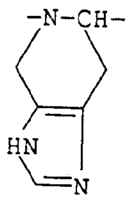

or $R_5$ is 2,3-dihydro-2-indanyl, $Y_5$ is H and $R_{1a}$ has independently one of the significance given for $R_{1b}$, and

$R_{1b}$ is an optionally protected side chain of the amino acid residue or thiocarbonyl analogue $A_1$ indicated above, or $A_1$ and $A_2$ form together a pseudo-dipeptide or a dipeptide mimetics residue,

X is a radical of formula (a) or (b) as defined above, and
$A_3$ is $-CH_2-X_1-(CO)_m-Y_6$ wherein $X_1$ is as defined above, m is 0 or 1 and $Y_6$ is a cycloaliphatic residue; tri-($C_{1-4}$alkyl)methyl; optionally ring substituted diphenylmethyl; optionally substituted aryl; or an optionally substituted mono-, di- or tricyclic heteroaryl residue
with the provisos that
i)$Y_1$ in $A_3$ is other than optionally substituted aryl or pyridyl when $A_1$ is other than an $\alpha$-hydroxy acid residue, $A_2$ is $-NH-CHR_3-CO-$ and $R_7$ in (a) or (b) is optionally esterified COOH,
ii)$Y_5$ in (aa) is in R configuration when it is the residue of an optionally protected side chain residue of an $\alpha$-amino acid, $Y_6$ is optionally substituted aryl and $R_7$ is optionally esterified COOH,
and the physiologically-hydrolysable and -acceptable esters or amides thereof when $R_7$ is $-CO_2H$,
in free form, in salt form or in the form of complexes.

**2.** A compound according to Claim 1 wherein $A_2$ is

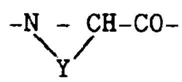

X is a radical of formula (a) as defined in Claim 1 and $A_3$ is $-CH_2-X_1-CO-Y_1$, $-CH_2-O-Y_2$ or $-CH_2-S-Y_3$ wherein Y, $Y_1$, $Y_2$, $Y_3$ and $X_1$ are as defined in Claim 1.

**3.** A compound according to Claim 1 wherein $A_1$ is an $\alpha$-hydroxy acid residue, X is a radical of formula (a) as defined in Claim 1, $A_2$ is $-NH-CHR_3-CO-$ and $A_3$ is $-CH_2-X_1-CO-Y_1$, $-CH_2-O-Y_2$ or $-CH_2-S-Y_3$ wherein $R_3$, $X_1$, $Y_1$ and $Y_2$ are as defined in Claim 1 and $Y_3$ is a cycloaliphatic residue or a 5-membered nitrogen-containing heterocyclic radical.

**4.** A compound according to Claim 1 wherein $A_1$ is other than an $\alpha$-hydroxy acid residue, $A_2$ is an $\alpha$-hydroxy acid residue, X is a radical of formula (a) as defined in Claim 1 and $A_3$ is $-CH_2-X_1-CO-Y_1$, $-CH_2-O-Y_2$ or $-CH_2-S-Y_3$ wherein $X_1$, $Y_1$, $Y_2$ and $Y_3$ are as defined in Claim 1.

**5.** A compound according to Claim 1 wherein $A_1$ and $A_2$ form together a radical of formula (aa) wherein $R_{1a}$ and $R_5$ form together $C_{2-5}$alkylene, $-CHY_5-CO-$ has the D configuration, X is a radical of formula (a) as defined in Claim 1 and $A_3$ is $-CH_2-X_1-(CO)_m-Y_6$ wherein $Y_6$ is optionally substituted aryl or pyridyl.

**6.** A compound according to Claim 1 wherein $A_1$ and $A_2$ form together a dipeptide mimetics residue.

**7.** A compound according to Claim 1 wherein $A_1$ and $A_2$ form together a dipeptide mimetics residue of formula $-NH-CHR_{1b}-W_1-$ wherein $R_{1b}$ is as defined in Claim 1 and $W_1$ is

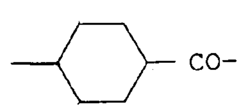 ;

a residue of formula $-NH-W_2-$ wherein $W_2$ is

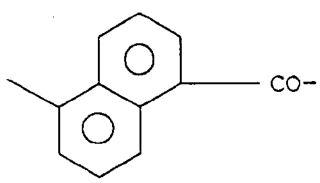 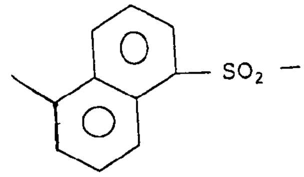 or 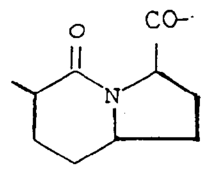

or a residue of formula -O-$W_3$- as defined in Claim 1.

**8.** A process for the preparation of a compound of formula I as defined in Claim 1, which process comprises:
a) removing at least one protecting group from a compound of formula I in protected form or adding a protecting group R at the terminal group of a compound of formula I; or
b) converting one compound of formula I into another compound of formula I; or
c) coupling together by an amide bond two peptide fragments, each of which contains at least one amino acid in protected or unprotected form and one peptide fragment containing a radical of formula (a) or (b) as defined in Claim 1, the peptide fragments being such that a protected or unprotected peptide having the sequence according to formula I above is obtained and, if necessary, removing the protecting group or groups from a compound of formula I in protected form; or
d) reacting a compound of formula II

$$R\text{-}A_1\text{-}A_2\text{-}X\text{-}CH_2\text{-}Z_a \qquad (II)$$

wherein R, $A_1$, $A_2$ and X are as defined in Claim 1, and $Z_a$ is a leaving group, with a compound of formula $HX_1$-$(CO)_m$-$Y_7$ wherein $X_1$ and m are as defined in Claim 1 and $Y_7$ is $Y_1$, $Y_2$, $Y_3$ or $Y_6$ or a functional derivative thereof when m is 1;
e) for the production of a compound of formula I wherein X is a radical of formula (a) or (b) monoalkylating or monoacylating a compound of formula III

$$R\text{-}A_1\text{-}A_2\text{-}NH\text{-}\underset{\displaystyle CH_2\text{-}R_{18}}{\underset{|}{CH}}\text{-}CHOH\text{-}CH_2OH \qquad (III)$$

wherein R, $A_1$ and $A_2$ are as defined in Claim 1 and $R_{18}$ is an ester group, with a compound $HX_1$-$(CO)_m$-$Y_7$ wherein m, $X_1$ and $Y_7$ are as defined above, or a functional derivative thereof when m is 1;
and recovering a compound of formula I thus obtained in free or salt form or in the form of a complex.

**9.** A compound of formula I according to any one of Claims 1 to 7, a physiologically-hydrolysable and -acceptable ester or amide thereof or a pharmaceutically acceptable salt thereof for use as a pharmaceutical.

**10.** A pharmaceutical composition comprising a compound of formula I according to any one of Claims 1 to 7, a physiologically-hydrolysable and -acceptable ester or amide thereof or a pharmaceutically acceptable salt thereof together with a pharmaceutically acceptable diluent or carrier therefor.